(19) Europäisches Patentamt
European Patent Office
Office européen des brevets

(11) Publication number: **0 457 671 A2**

(12) # EUROPEAN PATENT APPLICATION

(21) Application number: **91401243.0**

(51) Int. Cl.$^5$: **A61K 31/505**

(22) Date of filing: **14.05.91**

(30) Priority: **14.05.90 IT 4159890**

(43) Date of publication of application:
**21.11.91 Bulletin 91/47**

(84) Designated Contracting States:
**AT BE CH DE DK ES FR GB GR IT LI LU NL SE**

(71) Applicant: **FIDIA S.p.A.**
**Via Ponte della Fabbrica 3-A**
**I-35031 Abano Terme (Padova) (IT)**

(72) Inventor: **Della Valle, Francesco**
**Via Cerato 14**
**I-35100 Padova (IT)**
Inventor: **Romeo, Aurelio**
**Viale Ippocrate 93**
**I-00161 Rome (IT)**

(74) Representative: **Hirsch, Marc-Roger et al**
**Cabinet Hirsch 34 rue de Bassano**
**F-75008 Paris (FR)**

(54) **Dipyridamide for the treatment of diseases of the central or peripheral nervous system.**

(57) Use of dipyridamole or a pharmaceutically acceptable salt thereof for the treatment of disorders and diseases of the central or peripheral nervous system. This therapy is particularly useful for indications that require neurocyte repair or reproduction, such as pathologies of traumatic, compressive, degenerative or toxic-infective origin or in neurodegenerative diseases such as Alzheimer's or Parkinson's disease. The preferred mode of administration is by the oral route, suitably given in a daily dosage of active ingredient of from 3 to 10 mg/kg of body weight.

EP 0 457 671 A2

The present invention is directed to a new therapeutic use of dipyridamole, that is, 2,6-bis-(diethyl-ethanolamine)-4,8-dipiperidinopyrimido[5,4-d] pyrimidine of the formula:

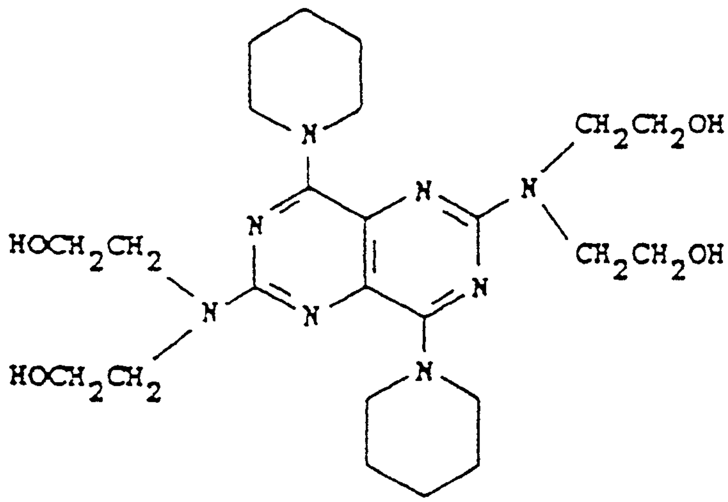

Dipyrimadole is a known coronary vasodilator (see for example UK Patent No. 807,826 (1959), Saraf. Seth. Indian J. Pharmacol. 15, 135 (1971), Takenaka et al, Arzneimittel-Forsch. 22, 892 (1972)), and is marketed under various trademarks (Anginal, Cardoxin, Curantyl, Persantine, etc.).

According to the present invention the said compound is also active on the central and peripheral nervous systems and can be used as a therapeutic agent to treat disorders and diseases of the nervous system. More precisely, dipyridamole is an efficacious agent in the regeneration and reproduction of neuritic cells which is a property that can be applied in many cases of impairment of the peripheral nervous system, and also in neuropathies of the central nervous system caused by or connected with deficiencies or alterations of substances responsible for nervous transmission. The action of dipyridamole is therefore similar to that of gangliosides and, thus, this compound can be used effectively in therapies in the same fields of application.

Some synthetic compounds, described in the literature, have a restorative action on the nerve cells of the central and/or peripheral nervous systems, for example, the pyrimidine derivatives of European patent application No. 257,102 dated 24.02.87, which have a very different structure from that of dipyridamole. Moreover, European patent application No. 169,405 dated 28.06.85 claims the use of compounds of the formula:

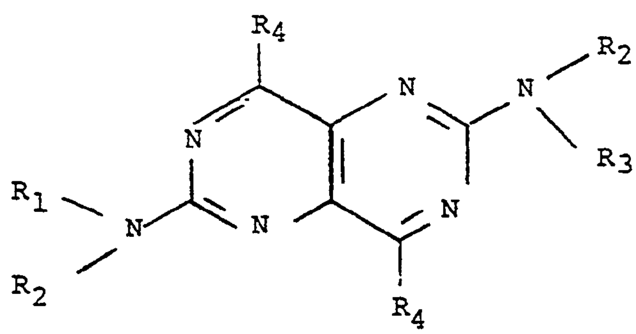

in which $R_1$ is a ($C_1$-$C_3$)-alkoxy-($C_2$-$C_3$)-alkyl radical, $R_2$ is a ($C_2$-$C_6$)-hydroxyalkyl radical or a $C_3$-dihydroxyalkyl radical, $R_3$ is a ($C_1$-$C_3$)-alkoxy-($C_2$-$C_3$)-alkyl radical or a $C_2$-$C_3$-hydroxyalkyl radical, $R_4$ is a dialkylamino radical, in which each radical has from 1 to 6 carbon atoms, or a diallyl- or di(methoxyethyl)-amino radical or a pyrrolinic, piperidinic, hexamethyleneamino, 1',2',5',6'-tetrahydropyridinic, morpholinic, thiomorpholinic or 1-oxidethiomorpholinic radical, which can also be substituted by a methyl group in therapy for cerebral insufficiency, for example, to increase cerebral potential, especially in cases of deficiency due to damage following trauma or alcohol abuse. These compounds were however already known (see for example German patent application No. 2300661) for their inhibiting action on platelet aggregation, as well as hyperemic, coronary-dilating and hypertensive action, properties also displayed by dipyridamole. The aforesaid formula also includes derivatives of dipyridamole, such as especially monoethers with aliphatic alcohols having from 1 to 3 carbon atoms (see the definition of $R_1$ above), but not dipyridamole itself.

It is surprising, according to the finding of the present invention, that this compound unexpectedly has a wide range of action on both the central and peripheral nervous systems.

In order to obtain the aforesaid therapeutic effects of dipyridamole, it is possible to use salts thereof obtained by acid addition, or double salts, especially salts of both these types which are able to produce gradual release of the active principle over time (retard or slow release forms). Examples of pharmaceutically acceptable addition salts are the hydrochloride and hydrobromide salts, sulfates, phosphates, acetates, maleates, succinates, lactates, tartrates, benzoates, citrates, gluconates, methanesulfonates, and p-toluenesulfonates.

The aforesaid vasodilating effect of dipyridamole does not constitute an obstacle to its therapeutic use according to the present invention. On the contrary, it represents a side effect which is by no means undesirable, and which can be avoided, if desired, by the administration of suitable preparations capable of limiting the vasodilating effect.

The present invention is therefore directed more precisely to the therapeutic use of dipyridamole or of one of its salts obtained by acid addition or of one of its double or complex salts or of a pharmaceutical preparation containing one or more of such compounds as the active ingredient for the treatment of nervous system disorders or diseases.

In particular, the invention is concerned with the therapeutic use of one of the aforesaid compounds in those cases which require neurocyte repair (neuronotrophic effect). Such cases may concern either the peripheral or the central nervous system. With regard to the peripheral nervous system, in particular pathologies of traumatic, compressive, degenerative or toxic-infective origin, in which it is necessary to stimulate nervous regeneration and the recovery of neuromuscular function, are to be considered. With regard to the central nervous system, the aforesaid products may be beneficial in neurodegenerative diseases, such as Alzheimer's or Parkinson's diseases, in the case of damage due to various kinds of acute insult of the nervous system, including trauma and postictal sequelae and in all cases of anomaly and impairment of neurotrophic function. The properties of inducing neuritic growth and enhancing neuronotrophic effect can be demonstrated by the following experiments on neuronal cell cultures.

MATERIALS AND METHODS

EXPERIMENTS IN VITRO

1a. Cell cultures of neuroblastoma from C 1300 mice, clone neuro-2a supplied by American Type Culture Collection (ATCC), were employed. The cells are seeded in an amount of 10,000 per 16 mm well, in a medium with 10% serum (3). The next day the medium is changed with an equal volume of fresh medium containing the product to be tested.

1b. Addition of dipyridamole to the cultures and the parameters assessed.

Dipyridamole is dissolved in a buffer with hydrochloric acid at a concentration of $1X10^{-2}M$ and then dissolved in the culture medium to obtain final concentrations of $1X10^{-6}$, $1X10^{-5}$, $2.5X10^{-5}$, $5X10^{-5}$ and $1X10^{-4}$. This is then added to the culture cells, the Ph having been adjusted, if necessary.

24 hours later the cultures are fixed and the percentage of neurite-bearing cells is counted.

2a. Dorsal root ganglia (DRG) are removed from chick embryos at day 8 of development (E8); the ganglia are then isolated and the cell suspension is enriched (4). The isolated neurons thus obtained are plated in wells containing polyornithine-laminin in DMEM (Dulbecco's Modified Eagle's Medium) containing 10% fetal calf serum (5).

2b. Addition of the dipyridamole compound and the parameters assessed.

Dipyridamole is solubilized in hydrochloric acid and buffer at a concentration of $10^{-2}M$ and then diluted with medium to obtain final concentrations of $1X10^{-4}M$, $5X10^{-5}M$, $1X10^{-5}M$ and $5X10^{-6}M$. It is then added to the cells and incubated for 48 hours in the presence of NGF (1 ng/ml). Neurite growth is assessed by antineurofilament antibody (RT 97; D.O.=492 nm) and ELISA.

RESULTS

The derivative dipyridamole proved to be very active in inducing neurite growth in $N_2A$ neuroblastoma cells following a dose-effect course with maximum activity between $2.5X10^{-5}M$ and $1X10^{-4}M$ (Table 1).

It is important to note that the derivative is active at very low doses and that it forms very long and branched neurites.

Moreover, on dorsal root ganglia cells from chick embryo at day 8 of development (DRG E8), dipyridamole promotes the formation of neurites with a dose-effect relationship (Table 2) and it notably enhances the effect of NGF (Table 3).

## CONCLUSION

Dipyridamole proved active in inducing neurito genesis on primary neuronal cell cultures and in enhancing the effect of NGF on chick embryo dorsal root ganglia.

Table 1

Effect of dipyridamole on neurite growth in $N_2A$ neuroblastoma cells: dose-effect relationship.

| | | % cells with neurites |
|---|---|---|
| Control | | 5 ± 2 |
| dipyridamole | $1X10^{-6}M$ | 5 ± 6 |
| | $1X10^{-5}M$ | 12 ± 5 |
| | $2.5X10^{-5}M$ | 37 ± 8 |
| | $5X10^{-5}M$ | 59 ± 4 |
| | $1X10^{-4}M$ | 56 ± 1 |
| $GM_1$ | $1X10^{-4}M$ | 36 ± 6 |

Morphology (No. of cells bearing neurites expressed as %) was assessed after 24 hrs of incubation at different concentrations of dipyridamole and expressed in terms of molarity.

Table 2

Effect of dipyridamole on DRG neurite outgrowth

| | | % increase |
|---|---|---|
| Dipyridamole | | |
| | $1X10^{-4}M$ | 0 |
| | $5X10^{-5}M$ | 4 |
| | $1X10^{-6}M$ | 37 |
| | $5X10^{-6}M$ | 38 |

DRG E8 cells are incubated for 48 hrs with $GM_1$ $1X10^{-4}M$ or dipyridamole at various concentrations (expressed in terms of molarity) and in the absence of NGF.

The effect of dipyridamole is assessed in terms of its increase compared to $GM_1$ and expressed as % (the effect of $GM_1$ is therefore expressed as zero).

Table 3

Enhancement of the effect of NGF on DRG.

| | | % increase |
|---|---|---|
| Dipyridamole | $1X10^{-4}M$ | 83 |
| | $5X10^{-5}M$ | 93 |
| | $1X10^{-5}M$ | 89 |
| | $5X10^{-6}M$ | 25 |
| $GM_1$ | $1X10^{-4}M$ | 32 |

The DRG E8 cells were incubated for 48 hours with NGF (1 ng/ml) ± dipyridamole or $GM_1$.

The % increase in effect was assessed in comparison to NGF.

The following references describe the above experimental procedures:

1. Leon, et al (1982): "Morphological and biochemical effect of gangliosides in neuroblastoma cells." Dev. Neurosci. 5: 108-14.

2. Skaper, et al (1983): "Ionic behaviours and Nerve Growth Factor dependence in developing chick ganglia. II Studies with neurons of dorsal root ganglia." Dev. Biol. 98: 257-264.

3. Leon, et al (1984): "Dorsal root ganglia and nerve growth factor: a model for understanding the mechan-

ism of $GM_1$ effect on neuronal repair." J. Neurosci. Res. 12: 277-87.

Dipyridamole is administered preferably in the form of pharmaceutical preparations in which it is the active ingredient, the excipients or carriers being those conventionally used in pharmaceutical preparations. Administration can be by the parenteral route, for example intravenous, subcutaneous or intramuscular, or by the intranasal, local, rectal and especially the oral route. The preparations or formulations can therefore be in solid or semisolid form, for example pills, tablets, gelatinous capsules, capsules, suppositories, or soft gelatin capsules, or in the form of solutions or freeze-dried powders to be mixed with one or more pharmaceutically acceptable excipients or diluents, suitable for the aforesaid uses and with an osmolarity which is compatible with physiological fluids. For local use, preparations in the form of creams or ointments for topical use, should be considered; for inhalant use, preparations in the form of sprays, for example nose sprays, should be considered.

The preparations of the invention can be used for administration to humans or animals. They contain preferably from 0.01% to 10% by weight of active component for solutions, sprays, ointments and creams and from 1% to 100%, and preferably from 5% to 50%, by weight of active compound for preparations in solid form. Dosages to be administered depend on individual needs, on the desired effect and on the administration route. For the oral route, the daily dosage varies preferably from about 3 to 10 mg/kg of body weight.

The following are examples of pharmaceutical preparations that can be employed in the present invention.

Preparation No. 1

Tablets containing 100 mg of dipyridamole:
1 tablet contains:

| | |
|---|---|
| Dipyridamole | 100.0 mg |
| Lactose | 70.0 mg |
| Starch | 40.0 mg |
| Polyvinylpyrrolidone | 8.0 mg |
| Magnesium stearate | 2.0 mg |
| TOTAL | 220.0 mg |

Preparation No. 2

Gelatine capsules containing 200 mg of dipyridamole:
1 capsule contains:

| | |
|---|---|
| Dipyridamole | 200.0 mg |
| Starch | 90.0 mg |
| Talc | 10.0 mg |
| TOTAL | 300.0 mg |

## Claims

1. Use of dipyridamole or a pharmaceutically acceptable salt thereof in the manufacture or preparation of a pharmaceutical formulation for the treatment of disorders and diseases of the nervous system.

2. The use of dipyridamole according to claim 1 for indications that require neurocyte repair or reproduction.

3. The use of dipyridamole according to claim 1 or claim 2 in central nervous system disorders.

4. The use of dipyridamole according to claim 1 or claim 2 in peripheral nervous system disorders.

5. The use of dipyridamole according to claim 4 in pathologies of traumatic, compressive, degenerative or

toxic-infective origin.

6. The use of dipyridamole according to claim 3 in neurodegenerative diseases.

7. The use of dipyridamole according to claim 6 in Alzheimer's disease.

8. The use of dipyridamole according to claim 6 in Parkinson's disease.

9. The use of dipyridamole according to claim 6 in cases of damage due to acute insult of the nervous system.

10. The use of dipyridamole according to claim 6 in anomalies and impairments of neuronotrophic function.

11. The use of dipyridamole according to any of claims 1-10 by the oral route.

12. The use of dipyridamole according to claim 11, in which a daily dosage of dipyridamole of from 3 to 10 mg/kg of body weight is administered.

13. The use of dipyridamole according to any of claims 1-12, in which said salt is obtained by acid addition.

14. The use of dipyridamole according to any of claims 1-12, in which said salt is a complex salt of dipyridamole.